# EUROPEAN PATENT SPECIFICATION

(11) **EP 3 773 136 B1**
(45) Date of publication and mention of the grant of the patent: **10.06.2026**
(21) Application number: 19715742.3
(22) Date of filing: 22.03.2019
(51) Int. Cl.: A61B 1/018, A61B 1/32, A61B 18/14, A61B 18/16, A61B 1/00

(54) **DEVICES FOR GROUNDING LUMINAL ELECTROSURGERIES**
VORRICHTUNGEN ZUR ERDUNG LUMINALER ELEKTROCHIRURGISCHER VORRICHTUNGEN
DISPOSITIFS POUR LA MISE À LA TERRE EN ÉLECTROCHIRURGIE INTRACAVITAIRE

(30) Priority: 29.03.2018 US 201862649901 P
(43) Date of publication of application: 17.02.2021
(73) Proprietor: Boston Scientific Scimed Inc., Maple Grove, Minnesota 55311 (US)
(72) Inventor: XU, Mingxiang, Wayland, Massachusetts 01775 (US); SMITH, Paul, Smithfield, Rhode Island 02917 (US); MCELWEE, Kevin J., Berwick, Maine 03901 (US)
(74) Representative: Vossius & Partner Patentanwälte Rechtsanwälte mbB
(86) International application number: PCT/US2019/023590
(87) International publication number: WO 2019/190915

(56) References cited:
- US-A1- 2009 062 790
- US-A1- 2012 157 987
- US-A1- 2013 274 553
- US-A1- 2015 272 564
- US-A1- 2017 296 260

## Description

### FIELD

The present disclosure relates generally to the field of medical devices and treating tissues within body passages. In particular, the present disclosure relates to endoluminal electrosurgical devices, systems and methods for providing treatment of tissue.

### BACKGROUND

Medical devices are often used to extract undesired and/or foreign material from the body. These medical devices use various extraction methods, such as dissection, coagulation, fulguration, ablation, etc., of undesired body matter. An exemplary procedure that uses such methods is electrosurgery. Electrosurgery involves the application of energy to biological tissue to cut, ablate, cauterize, coagulate, desiccate, and/or fulgurate tissue. Electrosurgery may use various types of high-frequency electrical energy to directly heat the tissue.

Generally, electrosurgery is performed using a radio frequency electrosurgical generator and a hand piece including one or two electrodes. A monopolar instrument comprises only one active electrode while a bipolar instrument includes two active electrodes at the surgical site, with one of the two active electrodes acting as ground electrode when the other electrode is active, and vice versa. The monopolar instrument requires the application of another instrument called a dispersive or return electrode elsewhere on or otherwise coupled to the patient's body to defocus or disperse the radio frequency current and returning the energy to the electrosurgical generator to prevent injury to the underlying tissue. However, often injury to the patient can occur from the energy traveling a large distance from the active electrode through the body to the return electrode. Also, doctors must be careful to avoid complications such as direct coupling, insulation failure, and capacitive coupling, among other potential complications that may result in patient injury.

A bipolar electrosurgical instrument typically includes forceps or other end effectors with the two tines of the forceps performing the active and return electrode functions. Only the tissue grasped by the forceps may be included in the electrical circuit. Bipolar instruments require less voltage than monopolar instruments, and since the active and return electrodes are at the site of surgery, the risk of accidental injury to the patient with bipolar instruments may be less compared to monopolar devices. Bipolar instruments perform well when sealing vessels, however they include lateral thermal spread that will continue until the device activation is ceased. Bipolar electrosurgical instruments are limited in their application and may provide less precise cutting compared to monopolar instruments. There is a need for a hybrid between monopolar and bipolar electrosurgical instruments that may utilize voltages similar to bipolar instruments and yet have similar cutting power to monopolar instruments.

US 2009/0062790 A1 relates to direct visualization bipolar ablation systems which utilize bipolar electrode arrangements. Such assemblies are configured to facilitate the application of bipolar energy delivery, such as RF ablation, to an underlying target tissue for treatment in a controlled manner while directly visualizing the tissue during the bipolar ablation process. US 2017/0296260 A1 relates to a device and methods of use to endoluminally ablate and/or occlude a body vessel using a radiofrequency ("RF") signal. The device has two conductive elements or electrodes that form various electrical circuits, and the two conductive elements are operable to create an electrical field. The electrical field ablates the body vessel, forming an occlusion.

In US 2012/0157987 A1 a catheter and catheter system for eccentric remodeling and/or removal of atherosclerotic material of a blood vessel of a patient includes an elongate flexible catheter body with a radially expandable structure. A plurality of electrodes or other electrosurgical energy delivery surfaces can radially engage atherosclerotic material when the structure expands. An atherosclerotic material detector near the distal end of the catheter body may measure circumferential atherosclerotic material distribution, and a power source selectively energizes the electrodes to eccentrically remodel the measured atherosclerotic material.

Conventional electrosurgical devices often have large profiles and may not be configured to be inserted into difficult to reach areas of the body or for use with endoscopic devices. A variety of advantageous medical outcomes may therefore be realized by the electrosurgical devices, systems, and methods of the present disclosure.

### SUMMARY

The invention is defined by the features of the independent claim. Any methods of treatment by surgery are not claimed as such, but are presented for illustrative purposes only.

In one aspect, the present disclosure relates to a surgical system comprising an elongate tubular body, which may include a proximal end, a distal end and one or more working channels extending therebetween. A working space expanding system may be positioned at a distal portion of the elongate tubular body. The working space expanding system may be movable between a non-expanded insertion position and an expanded position to form an expanded region. A surgical device may be disposed within a first working channel of the elongate tubular body. At least one return electrode may be disposed on an outer surface of the working space expanding system. The surgical system may include a power source configured to deliver energy to the surgical device, and in electrical communication with the at least one return electrode. The surgical device may be electrically connected to *(e.g.,* in electrical communication with) the power source by one or more conductive elements. The at least one return electrode may be electrically connected to the power source by one or more conductive elements. The surgical device may include an insulated portion and an uninsulated active (*e.g.*, performing) electrode. The working space expanding system may include first and second flexible members movable between the non-expanded position and the expanded position to form the expanded region. Each of the first and second flexible members may include an insulated portion and an uninsulated portion. The at least one return electrode may include a first return electrode disposed on the uninsulated portion of the first flexible member, and a second return electrode disposed on the uninsulated portion of the second flexible member. The first return electrode may be configured to contact an inner wall of a body lumen at a first location, and the second return electrode may be configured to contact the inner wall of the body lumen at a second location, when the first and second flexible members move to the expanded position. The surgical device may be configured to contact the inner wall of the body lumen at a third location to deliver energy from the power source, through a portion of the tissue of the body lumen, and to the first and second return electrodes. The energy delivered from the surgical device may manipulate, treat or otherwise affect, tissue of the body lumen. A cover may be disposed around a portion of the working space expanding system. The at least one return electrode may include a first return electrode disposed on an outer surface of the cover at first location, and a second return electrode disposed on the outer surface of the cover at a second location. The cover may include an opening opposite the first and second return electrodes. The first flexible member may be disposed adjacent to a first side of the opening in the cover, and the second flexible member may be disposed adjacent to a second side of the opening in the cover. The first return electrode may be configured to contact an inner wall of a body lumen at the first location, and the second return electrode may be configured to contact the inner wall of the body lumen at the second location, when working space expanding system *(e.g.,* the first and second flexible members) move to the expanded position. The surgical device may be configured to contact the inner wall of the body lumen at a third location to deliver energy from the power source, through a portion of the tissue of the body lumen, and to the first and second return electrodes. The energy delivered from the surgical device may manipulate, treat or otherwise affect, tissue of the body lumen. A distal portion of the working space expanding system may include an endcap, and the at least one return electrode may be disposed on an outer surface of the endcap. The endcap may be configured to contact an inner wall of a body lumen as the working space expanding system *(e.g.,* first and second flexible members) move to the expanded position, to place the at least one return electrode in contact with the inner wall of a body lumen at a first location. A sleeve may be disposed around a distal portion of the elongate tubular body, and the at least one return electrode may be disposed on an outer surface of the sleeve. The at least one return electrode may be configured to contact an inner wall of a body lumen at a first location when the working space expanding system *(e.g.,* the first and second flexible members) move to the expanded position. The surgical device may be configured to contact an inner wall of the body lumen at a second location to deliver energy from the power source, through a portion of the tissue of the body lumen, and to the at least one return electrode. The energy delivered from the surgical device may manipulate, treat or otherwise affect, a tissue of the body lumen.

In another aspect, the present disclosure relates to a surgical system comprising an elongate tubular body, which may include a proximal end, a distal end and one or more working channels extending therebetween. A working space expanding system may be positioned at a distal portion of the elongate tubular body. The working space expanding system may be movable between a non-expanded insertion position and an expanded position to form an expanded region. A surgical device may be disposed within a first working channel of the elongate tubular body. At least one return electrode may be disposed on an outer surface of the working space expanding system. The surgical system may include a power source configured to deliver energy to the surgical device, and in electrical communication with the at least one return electrode. The surgical device may be electrically connected to *(e.g.,* in electrical communication with) the power source by one or more conductive elements. The at least one return electrode may be electrically connected to the power source by one or more conductive elements. The surgical device may include an insulated portion and an uninsulated active (*e.g.*, performing) electrode. The working space expanding system may include first and second flexible members movable between the non-expanded position and the expanded position to form the expanded region. Each of the first and second flexible members may include an insulated portion and an uninsulated portion. The at least one return electrode may include a first return electrode disposed on the uninsulated portion of the first flexible member, and a second return electrode disposed on the uninsulated portion of the second flexible member. The first return electrode may be configured to contact an inner wall of a body lumen at a first location, and the second return electrode may be configured to contact the inner wall of the body lumen at a second location, when the working space expanding system *(e.g.,* first and second flexible members) moves to the expanded position. The surgical device may be configured to contact the inner wall of the body lumen at a third location to deliver energy from the power source, through a portion of the tissue of the body lumen, and to the first and second return electrodes. The energy delivered from the surgical device may manipulate, treat or otherwise affect, a tissue of the body lumen. A cover may be disposed around a portion of the working space expanding system. The at least one return electrode may include a first return electrode disposed on an outer surface of the cover at a first location, and a second return electrode disposed on the outer surface of the cover at a second location. The first return electrode may be configured to contact an inner wall of a body lumen at the first location, and the second return electrode may be configured to contact the inner wall of the body lumen at the second location, when the working space expanding system *(e.g.,* first and second flexible members) moves to the expanded position. The cover may include an opening opposite the first and second return electrodes. The first flexible member may be disposed adjacent to a first side of the opening in the cover, and the second flexible member may be disposed adjacent to a second side of the opening in the cover. The surgical device may be configured to contact the inner wall of the body lumen at a third location to deliver energy from the power source, through a portion of the tissue of the body lumen, and to the first and second return electrodes. The energy delivered from the surgical device may manipulate, treat or otherwise affect, a tissue of the body lumen. A distal portion of the working space expanding system may include an endcap, and the at least one return electrode may be disposed on an outer surface of the endcap. The endcap may be configured to contact an inner wall of a body lumen as the first and second flexible members move to the expanded position, to place the at least one return electrode in contact with the inner wall of a body lumen at a first location. The energy delivered from the surgical device may manipulate, treat or otherwise affect, a tissue of the body lumen.

In yet another aspect, the present disclosure relates to a surgical system comprising an elongate tubular body, which may include a proximal end, a distal end and one or more working channels extending therebetween. A working space expanding system may be positioned at a distal portion of the elongate tubular body. The working space expanding system may movable between a non-expanded insertion position and an expanded position to form an expanded region. A surgical device may be disposed within a first working channel of the elongate tubular body. The surgical device may include an insulated portion and an uninsulated active (*e.g.*, performing) electrode. At least one return electrode may be disposed on an outer surface of the working space expanding system. The surgical system may include a power source configured to deliver energy to the surgical device, and in electrical communication with the at least one return electrode. A sleeve may be disposed around a distal portion of the elongate tubular body, and the at least one return electrode may be disposed on an outer surface of the sleeve. The working space expanding system may include first and second flexible members movable between the non-expanded position and the expanded position to form the expanded region. The at least one return electrode may be configured to contact an inner wall of a body lumen at a first location when the first and second flexible members move to the expanded position. The surgical device may be configured to contact an inner wall of the body lumen at a second location to deliver energy from the power source, through a portion of the tissue of the body lumen, and to the at least one return electrode. The energy delivered from the surgical device may manipulate, treat or otherwise affect, tissue of the body lumen.

In yet another aspect, the present disclosure relates to a surgical system comprising an elongate tubular body, which may include a proximal end, a distal end and one or more working channels extending therebetween. The surgical system may include an elongate insulated sheath configured to extend over at least a portion of an outer surface of the elongate tubular body. An expandable member may be positioned at a distal portion of the insulated sheath. The expandable member may be configured to move between a non-expanded insertion position and an expanded position to form an expanded region. A surgical device may be movably disposed within a first working channel of the elongate tubular body. The surgical device may include an insulated portion and an uninsulated active (*e.g.*, performing) electrode. The expandable member may include an opening into/through which a distal portion of the surgical device may extend. At least one return electrode may be disposed on an outer surface of the expandable member. The surgical system may include a power source configured to deliver energy to the surgical device, and in electrical communication with the at least one return electrode. The at least one return electrode may be configured to contact an inner wall of a body lumen at a first location when the expandable member moves to the expanded position. The surgical device may be configured to contact an inner wall of the body lumen at a second location to deliver energy from the power source, through a portion of the tissue of the body lumen, and to the at least one return electrode. The energy delivered from the surgical device may manipulate, treat or otherwise affect, tissue of the body lumen.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. In the figures:
FIGS. 1A-1D provide perspective views of a medical device, according to one embodiment of the present disclosure.
FIG. 2 provides a perspective view of a medical device, according to one embodiment of the present disclosure.
FIG. 3 provides a perspective view of a medical device, according to one embodiment of the present disclosure.
FIG. 4 provides a perspective view of a medical device, according to one embodiment of the present disclosure.
FIG. 5 provides a perspective view of a medical device, according to one embodiment of the present disclosure.

### DETAILED DESCRIPTION

The present disclosure is not limited to the particular embodiments described. The terminology used herein is for the purpose of describing particular embodiments only, and is not intended to be limiting beyond the scope of the appended claims. Unless otherwise defined, all technical terms used herein have the same meaning as commonly understood by one of ordinary skill in the art to which the disclosure belongs.

Although embodiments of the present disclosure are described with specific reference to medical devices and systems configured to be inserted into a body lumen of a patient for intraluminal treatment of tissues, *e.g.*, within the gastrointestinal tract, it should be appreciated that such medical devices and systems may be used in a variety of medical procedures within various luminal body passages, including, by way of non-limiting example, the vascular system, pulmonary system, respiratory system, urogenital system, upper GI tract, and the like. The devices and system can be inserted via different access points and approaches, *e.g.*, percutaneously, endoscopically, laparoscopically, or some combination thereof.

As used herein, the singular forms "a," "an," and "the" are intended to include the plural forms as well, unless the context clearly indicates otherwise. It will be further understood that the terms "comprises" and/or "comprising," or "includes" and/or "including" when used herein, specify the presence of stated features, regions, steps, elements and/or components, but do not preclude the presence or addition of one or more other features, regions, integers, steps, operations, elements, components and/or groups thereof.

As used herein, the term "distal" refers to the end farthest away from the medical professional when introducing a device into a patient, while the term "proximal" refers to the end closest to the medical professional when introducing a device into a patient.

In various embodiments, the present disclosure relates to intraluminal surgical systems and related methods for providing treatment energy to body tissues. For example, the surgical systems disclosed herein generally relate to luminal surgical devices configured to perform monopolar or bipolar electrosurgical procedures within a variety of body lumens or passageways.

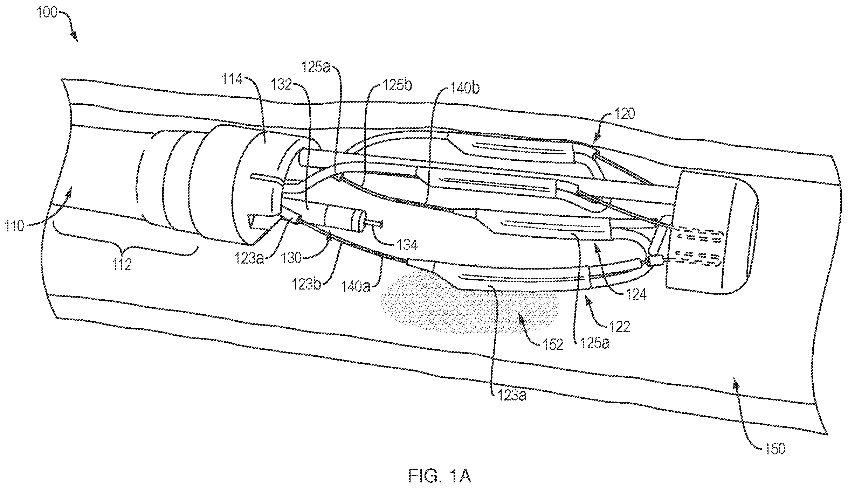

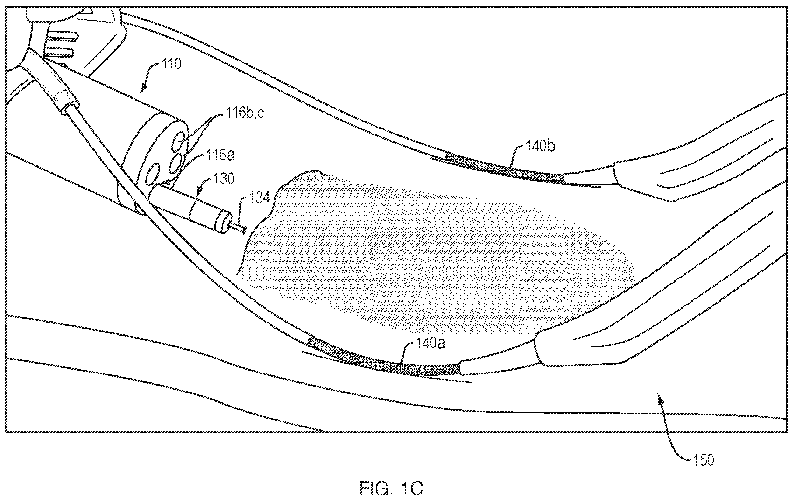

Referring to FIG. 1A, in one embodiment, a surgical system 100 of the present disclosure may include a flexible elongate tubular body 110 *(e.g.,* endoscope, gastroscope, colonoscope, delivery catheter, or other device for delivering a medical tool to a treatment site) comprising a proximal end (not shown), a distal end 114 and one or more working channels 116a-c extending therebetween (FIGS. 1C-1D). A working space expanding system 120 may be attached to, and extend distally beyond, a distal portion 112 of the elongate tubular body 110. The working space expanding system 120 may be configured to move between a non-expanded *(e.g.,* unexpanded, collapsed, etc.) insertion position, and an expanded *(e.g.,* unconstrained, deployed, etc.) position to form an expanded region within a body lumen of patient. A surgical device 130 *(e.g.,* electrosurgical device, surgical catheter, first electrode, cutting electrode, end effector, etc.) may be movably *(e.g.,* slidably, rotatably, etc.) disposed within a first working channel 116a of the elongate tubular body 110. The surgical device 130 may include an insulated outer portion 132 and an uninsulated *(e.g.,* conductive) active electrode portion 134. For example, the insulated outer portion 132 may extend along a length of the surgical device 130 extending through the first working channel 116a, and active electrode 134 may extend through/within the insulated outer portion 132. A distal end *(e.g.,* distal tip) of the active electrode 134 may extend distally beyond the insulated outer portion 132 of the surgical device 130, *e.g.,* into the working space expanding system 120.

At least one return electrode *(e.g.,* second electrode, grounding electrode, intraluminal grounding element, etc.) may be attached to, or otherwise disposed on or along *(e.g.,* integrally formed with, etc.) an outer surface of the working space expanding system 120. For example, in one embodiment, the working space expanding system 120 may include first and second flexible members 122, 124 configured to move *(e.g.,* bow, flex or bend, etc.) between the non-expanded insertion position and the expanded position to form the expanded region. The first and second flexible members 122, 124 may each include an insulated portion 123a, 125a, and an uninsulated portion 123b, 125b. A first return electrode 140a may be disposed on or along an outer surface of the uninsulated portion 123b of the first flexible member 122, and a second return electrode 140b may be disposed on or along an outer surface of the uninsulated portion 125b of the second flexible member 124.

In various embodiments, the first and second flexible members 122, 124 may extend along an inner and/or outer surface of the elongate tubular body 110, such that a proximal portion (not shown) of each flexible member may be actuated by a medical professional, *e.g*., using a suitable handle, actuator or the like. Similarly, the surgical device 130 may extend through an entire length of the elongate tubular body 110, such that a proximal portion (not shown) of the surgical device 130 may be actuated by a medical professional, *e.g.*, using a suitable handle, actuator or the like. The insulated portions 123a, 125a may extend along an entire length of the respective first and second flexible members 122, 124 along and/or through the elongate tubular body 110.

The surgical system 100 may further include a power source (not shown) configured to transmit or deliver energy to the active electrode 134 of the surgical device 130, and in electrical communication with the return electrode(s) 140a, 140b. In various embodiments, the power source may be incorporated within a portion of the elongate tubular body 110, *e.g.,* at or near the proximal end (not shown). Alternatively, the power source may include an external unit or module, *e.g.,* a standalone device, electrically connected to surgical system 100. The power source may supply any suitable energy, such as electrical, laser, thermal, ultrasound, etc. For example, in one embodiment, the power source may generate radiofrequency energy. The power source may include a controller and a user interface with various components, such as processors for processing instructions (e.g. program instructions), memory, and user input devices. In various embodiments, the controller and the user interface may modulate the characteristics of the energy supplied to the surgical system 100. The user interface may display the energy output of the surgical device, and/or may display an image of the body lumen and/or lesion, such as an image from an imaging sensor present at the distal tip of the elongate tubular body 110. One or more actuation mechanisms, such as buttons, dials, sensors, *etc.*, may be present on the power source, the controller and/or the user interface. In various examples, a medical professional may adjust the energy output of the surgical device via one or more actuators on the power source, the controller and/or the user interface.

In one embodiment, a proximal end (not shown) of the active electrode 134 of the surgical device 130 may be electrically connected to the power source. In addition, the first and second return electrodes 140a, 140b, may be electrically connected to the power source by the conductive materials which comprise the first and second flexible members 122, 124 extending extend along an inner and/or outer surface of the elongate tubular body 110.

Still referring to FIG. 1A, in use and by way of example, the surgical system 100 may be advanced through a body lumen 150 of a patient in the non-expanded insertion position to the site of a known or suspected tissue lesion 152 within a tissue wall of the body lumen 150. The surgical system may be oriented *(e.g.,* rolled, twisted, etc.) such that the first and second flexible members 122, 124 are positioned on opposite sides of a tissue lesion 152.

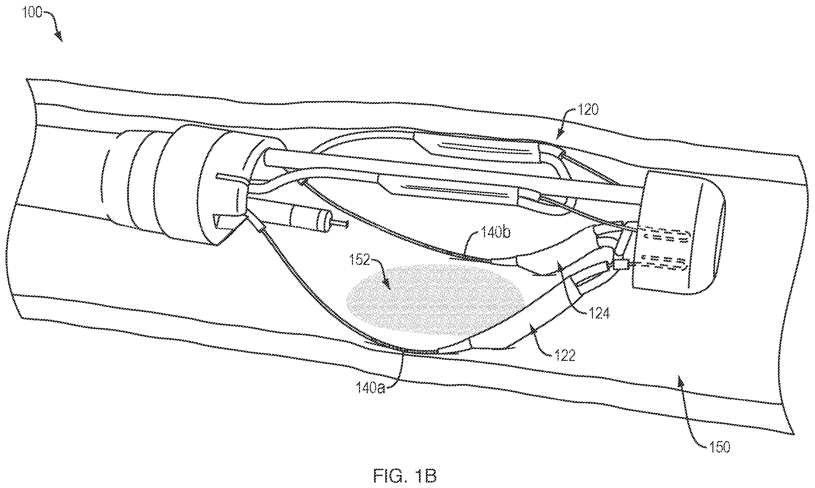

Referring to FIG. 1B, the first and second flexible members 122, 124 may then be actuated, *e.g.,* from a handle or actuator at a proximal end thereof, as discussed above, such that the first and second flexible members 122, 124 bow or flex outward to form the expanded region of the working space expanding system 120. In the expanded position, the first and second flexible members 122, 124 may apply radial outward force to at least a portion of the tissue wall of the body lumen 150 such that the first and/or second return electrodes 140a, 140b are placed in firm contact *(e.g.,* direct contact, intimate contact, etc.) with the tissue wall of the body lumen 150 at first and second locations, respectively. Although the lesion 152 of FIG. 1B is depicted as fitting within the space between the first and second flexible members 122, 124, in various embodiments the lesion 152 may include a variety of different sizes and/or shapes. In addition, or alternatively, the first and second flexible members 122, 124 are not strictly required to contact respective portions of the tissue wall on opposite sides of the tissue lesion 152, but may contact any portion of the tissue wall in the general vicinity of the tissue lesion 152. In various embodiments, in addition to placing the first and second return electrodes 140a, 140b in firm contact with the tissue wall of the body lumen 150, in order to facilitate electrosurgical manipulation, or treatment of the tissue lesion (as discussed below), the expanded region of the working space expanding system 120 may also provide an increased working space within which one or more tools or other medical instruments may operate.

Referring to FIG. 1C, with the first and second return electrodes 140a, 140b placed in firm contact with the tissue wall of the body lumen 150, the surgical device 130 may be advanced within/through the first working channel 116a of the elongate tubular body 110 such that the active electrode 134 is placed in contact with the tissue wall of the body lumen 150 at a third location.

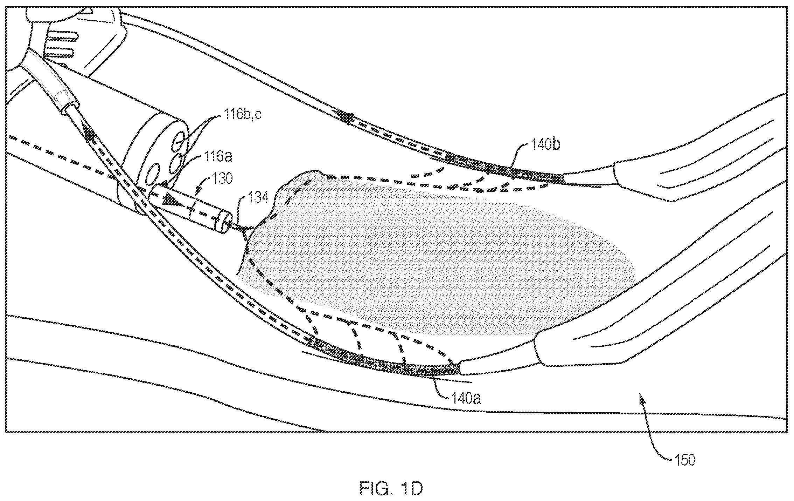

Referring to FIG. 1D, with the first and second return electrodes 140a, 140b, and the active electrode 134 of the surgical device 130, in contact with their respective first, second and third portions of the tissue wall of the body lumen 150, energy may be delivered from the power source to the surgical device 130. As indicated by the dashed-lines, the energy may then be locally emitted from the active electrode 134 of the surgical device, into and through a portion of the tissue wall of the body lumen 150, and received by the first and second return electrodes 140a, 140b. The energy may then be returned to the first and second return electrodes 140a, 140b to the power source, thereby preventing energy from travelling through other portions of the body lumen and/or patient's body. The energy emitted from the active electrode 134 of the surgical device may then be used to electrosurgically manipulate the tissue wall of the body lumen to remove or otherwise treat *(e.g.,* mark, cut, dissect, resect, coagulate, ablate, etc.), the tissue lesion 152.

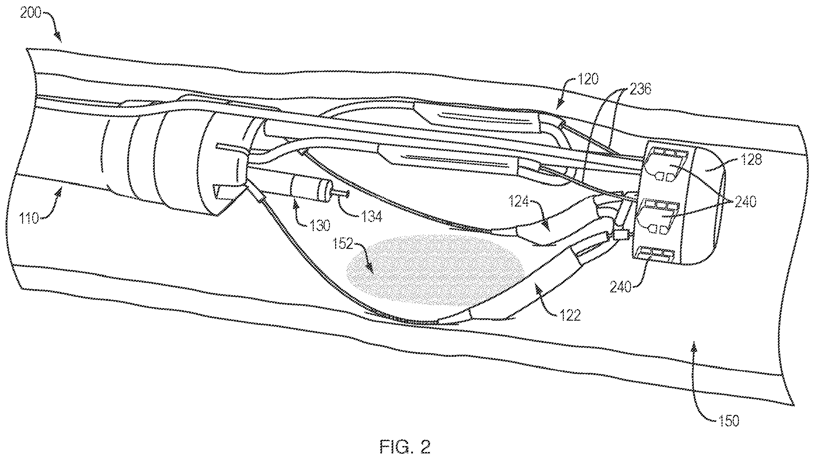

Referring to FIG. 2, in one embodiment, a surgical system 200 of the present disclosure may include similar or identical features of the surgical system 100, with the exception that the at least one return electrode is disposed on an endcap 128 positioned at or near the distal end of the working space expanding system 120. For example, the endcap 128 may provide a distal nexus at which a distal end of the first and second flexible members 122, 124 may be joined or connected. In one embodiment, the at least one return electrode may include a plurality of return electrodes 240 disposed uniformly or non-uniformly around a full or partial circumference of an outer surface of the endcap 128. One or more conductive elements 236 (*e.g.*, conductive wires) may extend along an inner or outer surface of the working space expanding system and/or elongate tubular body 110 to electrically connect each of the return electrodes 240 to the power source. In various embodiments, the endcap 128 may include an outer dimension configured to place at least one of the return electrodes 240 in firm contact with a tissue wall of the body lumen 150. For example, as the first and second flexible members 122, 124 move from the non-expanded insertion position to the expanded position to form the expanded region, as discussed above, the endcap 128 may deflect in an opposite direction within the body lumen 150, *e.g.,* toward a portion of the tissue wall opposite the lesion 152. With the return electrodes 240 of the endcap 128 in contact with the tissue wall of the body lumen, the surgical device 130 may be advanced and placed in contact with a separate portion of the tissue wall, as discussed above. The energy emitted from the active electrode 134 of the surgical device 130 may then be used to manipulate the tissue wall of the body lumen to remove or otherwise treat *(e.g.,* mark, cut, dissect, resect, coagulate, ablate, etc.) the tissue lesion 152, as discussed above.

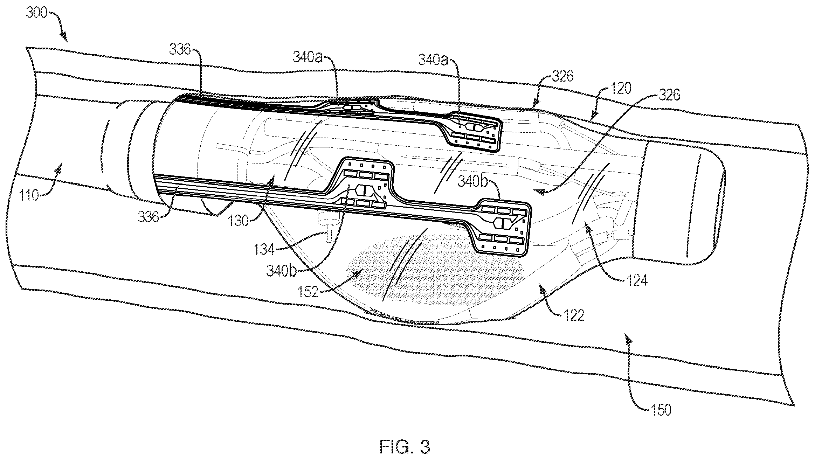

Referring to FIG. 3, in one embodiment, a surgical system 300 of the present disclosure may include similar or identical features of the surgical system 100, with the exception that the at least one return electrode is disposed on or along a cover 326 extending around (*e.g.*, partially enclosing) the working space expanding system 120. For example, one or more first return electrode 340a may be disposed on an outer surface of the cover 326 at a first location, and one or more second return electrodes 340b may be disposed on an outer surface of the cover at a second location, *e.g.*, adjacent to the first location. In various embodiments, the first and second return electrodes 340a, 340b may be permanently attached to *(e.g.,* wedged, swaged, painted, glued, embedded, etc.), or integrally formed with, the cover 326 using a suitable glue, adhesive, resin, solder, or other bonding techniques, as are commonly known in the art. One or more conductive elements 336 *(e.g.,* conductive wires) may extend along an inner or outer surface of the elongate tubular body 110 to electrically connect the first and second return electrodes 340a, 340b to the power source. In various embodiments, the cover 326 may include an outer dimension (*e.g.*, when the working space expanding system is in the expanded position) configured to place one or more of the first and/or second return electrodes 340a, 340b in firm contact with a tissue wall of the body lumen 150. For example, as the first and second flexible members 122, 124 move from the non-expanded insertion position to the expanded position to form the expanded region, all or a portion of the cover 326 may deflect in an opposite direction within the body lumen, *e.g.*, toward a portion of the tissue wall opposite the lesion 152. In one embodiment, a portion of the cover 326 opposite the first and second return electrodes 340a, 340b may include an opening through which the surgical device 130 (and accessory tools, discussed below) may access the tissue wall of the body lumen 150 and/or tissue lesion 152. For example, in one embodiment, the first flexible member 122 may be disposed along or adjacent to a first side of the opening of the cover 326, and the second flexible member 124 may be disposed along or adjacent to a second side of the opening of the cover 326.

With the first and second return electrodes 340a, 340b on the outer surface of the cover 326 in contact with the tissue wall of the body lumen 150, the surgical device 130 may be advanced and placed in contact with a separate portion of the tissue wall, as discussed above. The energy emitted from the active electrode 134 of the surgical device 130 may then be used to manipulate the tissue wall of the body lumen to remove or otherwise treat *(e.g.,* mark, cut, dissect, resect, coagulate, ablate, etc.) the tissue lesion 152, as discussed above.

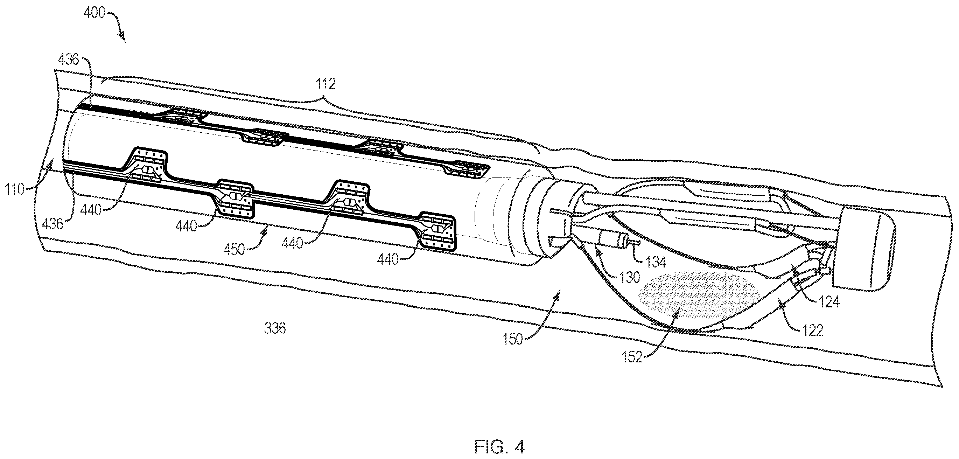

Referring to FIG. 4, in one embodiment, a surgical system 400 of the present disclosure may include the identical features of the surgical system 100, with the exception that the at least one return electrode may be disposed on or along a sleeve 450 extending around a distal portion 112 of the elongate tubular body 110. For example, the at least one return electrode may include a plurality of return electrodes 440 disposed uniformly or non-uniformly around a full or partial circumference of an outer surface of the sleeve 450. One or more conductive elements 436 may extend along an inner or outer surface of the elongate tubular body 110 to electrically connect each of the return electrodes 440 to the power source.

In various embodiments, the plurality of return electrode(s) 440 may be permanently attached to *(e.g.,* wedged, swaged, painted, embedded), or integrally formed with, the outer surface of the sleeve 450 using a suitable glue, adhesive, resin, solder, or other bonding techniques, as are commonly known in the art. In addition, or alternatively, an outer surface of the sleeve 450 may be formed from or include one or more conductive materials such that the entire sleeve 450 may serve as the return electrode. One or more conductive elements (not shown), (*e.g.*, conductive wires) may extend along an inner or outer surface of the elongate tubular body 110 to electrically connect each return electrode(s) to the power source.

In various embodiments, the sleeve 450 may include an outer dimension configured to place at least one of the return electrodes 440 in firm contact with a tissue wall of the body lumen 150. For example, as the first and second flexible members 122, 124 move from the non-expanded insertion position to the expanded position to form the expanded region, as discussed above, at least a portion of the sleeve 450 may deflect in an opposite direction within the body lumen 150, *e.g.,* toward a portion of the tissue wall opposite the lesion 152. In other embodiments, the sleeve 450 may be configured to expand or inflate within the body lumen 150 to place at least one of the return electrodes 440 in firm contact with a tissue wall of the body lumen. For example, one or more inflation/deflation lumens (not shown) may run along an inner or outer surface of the elongate tubular body 110 to deliver a suitable inflation medium (*e.g.*, normal saline, a biologically inert gas, etc.) into a lumen of the sleeve 450 to move the sleeve from a first non-expanded position to a second expanded position.

With the return electrodes 440 on the outer surface of the sleeve 450 in contact with the tissue wall of the body lumen 150, the surgical device 130 may be advanced and placed in contact with a separate portion of the tissue wall, as discussed above. The energy emitted from the active electrode 134 of the surgical device may then be used to manipulate the tissue wall of the body lumen to remove or otherwise treat *(e.g.,* mark, cut, dissect, resect, coagulate, ablate, etc.) the tissue lesion 152, as discussed above.

Although the working space expanding system 120 of FIGS. 1A-4 is generally depicted as including first and second flexible members 122, 124 configured to bow, flex or bend in a substantially "U-shaped" configuration, in various embodiments, the working space expanding system may include a single flexible member, or more than two flexible members *(e.g.,* three or more) configured to move to a variety of different shapes when in the non-expanded configuration. By way of non-limiting example, such flexible members may move to a "T-shaped" configuration, an "L-shaped" configuration, a circular or oval shaped configuration, and various symmetric or asymmetric variations thereof. Similarly, the number, arrangement and/or orientation of the return electrodes disposed on or along such flexible member(s) is not limited to the configuration of FIGS. 1A-4, but may vary.

In various embodiments, the working space expanding system may include proximal and distal balloons expandable to form the expanded regions. An outer surface of either (or both) of the proximal and distal balloons may include one or more return electrodes in electrical communication with a power source, and configured to be placed in firm contact with the tissue wall of the body lumen, in order to facilitate electrosurgical manipulation, or treatment of the tissue lesion (as discussed above). In addition, the proximal and/or distal balloons may expand to provide an increased working space within which one or more tools or other medical instruments may operate.

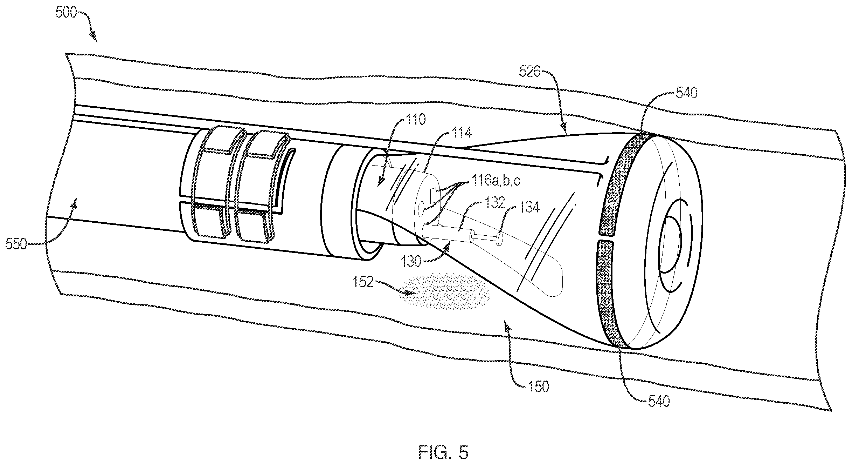

Referring to FIG. 5, in one embodiment, a surgical system 500 of the present disclosure may include a flexible elongate tubular body 110 *(e.g.,* endoscope, gastroscope, colonoscope, delivery catheter, etc.) comprising a proximal end (not shown), a distal end 114 and one or more working channels 116a-c extending therebetween. An elongate insulated sheath 550 may extend over and along all or a portion of an outer surface of the elongate tubular body 110. An expandable member 526 *(e.g.,* balloon, etc.) may be attached to and extend distally beyond a distal end of the insulated sheath 550. In various embodiments, an inflation/deflation lumen (not shown) may extend along an inner or outer surface of the insulated sheath 550, and may be configured to deliver a suitable inflation fluid/medium into an interior region of the expandable member 526. The expandable member 526 may be configured to move between a non-expanded *(e.g.,* collapsed, delivery, etc.) insertion position and an expanded position to form an expanded region, *e.g.*, by introducing or removing the inflation fluid from within the interior region of the expandable member 526. In addition, or alternatively, the expandable member 526 may be configured to move between the non-expanded and expanded positions using a mechanical, rather than fluidic, actuation mechanism, *e.g.*, one or more expandable/collapsible arms, collapsible/expandable framework, etc. (not shown).

A surgical device 130 *(e.g.,* first electrode, cutting electrode, end effector, etc.) may be movably (*e.g.*, slidably, rotatably, etc.) disposed within a first working channel 116a of the elongate tubular body 110. The surgical device 130 may include an insulated outer portion 132 and an uninsulated (*e.g*., conductive) active electrode portion 134. For example, the insulated outer portion 132 may extend along a length of the surgical device 130 extending through the first working channel 116a, and the active electrode 134 may extend through/within the insulated outer portion 132. A distal end *(e.g.,* distal tip) of the active electrode 134 may extend distally beyond the insulated outer portion 132 of the surgical device 130, *e.g.,* into an interior portion of the expandable member 526.

At least one return electrode may be attached to, or otherwise disposed on or along (*e.g.*, integrally formed with, etc.) an outer surface of the expandable member 526. For example, the at least one return electrode may include a plurality of return electrodes 540 uniformly or non-uniformly disposed around a full or partial circumference of a distal end of the expandable member 526. The surgical system 500 may further include a power source, as discussed above (not shown), configured to transmit or deliver energy to the surgical device 130, and in electrical communication with the plurality of return electrodes 540, as discussed above.

In use and by way of example, the surgical system 500 may be advanced through a body lumen 150 of a patient in the non-expanded insertion position to the site of a known or suspected tissue lesion 152 within a tissue wall of the body lumen 150. The expandable member 526 may then be moved to the expanded (*e.g.*, inflated) position, thereby applying radial outward force to at least a portion of the tissue wall of the body lumen 150 such that one or more of the plurality of return electrodes 540 are placed in firm contact *(e.g.,* direct contact, intimate contact, etc.) with the tissue wall of the body lumen 150. In various embodiments, in addition to placing the plurality of return electrodes 540 in firm contact with the tissue wall of the body lumen 150, *e.g.,* to facilitate electrosurgical manipulation of the tissue lesion (as discussed below), the expandable member 526 may also provide an increased working space within which one or more tools or other medical instruments may operate.

With one or more of the return electrodes 540 placed in firm contact with the tissue wall of the body lumen 150, the surgical device 130 may be advanced within/through the first working channel 116a of the elongate tubular body 110 such that the active electrode 134 extends through an opening in the expandable member 526 and is placed in contact with the tissue wall of the body lumen 150. With one or more of the return electrodes 540, and the active electrode 134 of the surgical device 130, in contact with their respective portions of the tissue wall of the body lumen 150, energy may be delivered from the power source to the surgical device 130. The energy may then be locally emitted from the active electrode 134 of the surgical device, into and through a portion of the tissue wall of the body lumen 150, and received by one or more of the return electrodes 540, as discussed above. The energy may then be returned from the plurality of return electrodes to the power source. The energy emitted from the active electrode 134 of the surgical device may then be used to electrosurgically manipulate the tissue wall of the body lumen to remove or otherwise treat *(e.g.,* mark, cut, dissect, resect, coagulate, ablate, etc.) the tissue lesion 152, as discussed above.

In various embodiments, any of the surgical systems 100, 200, 300, 400, 500, disclosed herein may operate in a monopolar mode or a bipolar mode. In particular, the power source may include a monopolar mode or bipolar mode. In some examples, the bipolar mode may use lower voltages while the monopolar mode may require higher voltages. Since the surgical systems of the present disclosure generally include one or more return electrodes positioned in the general proximity of the site of treatment within the body lumen 150, *e.g.,* at or near the lesion 152, the power density away from the treatment site may be lower than a conventional monopolar electrosurgery, *e.g.,* in which the return electrode(s) is/are positioned somewhere on the patient's exterior skin, typically not proximate to the site of surgery. By positioning the one or more return electrodes proximate to the target lesion 152, the surgical systems of the present disclosure may have similar efficiency and effectiveness as conventional monopolar electrosurgical techniques while outputting significantly lower energy levels and with a much lower risk of unwanted thermal damage to collateral tissue.

In addition, the ability of the surgical systems of the present disclosure to mitigate thermal damage by directing the electrosurgical energy superficially and subcutaneously (*e.g.*, endoluminally) may allow the active electrode 134 to include a smaller size or profile, thereby providing the medical professional with more refined control while manipulating, treating or otherwise affecting tissues of a body lumen.

In addition, the ability of the surgical systems of the present disclosure to mitigate thermal damage to collateral tissue by locally deploying the active electrode and the return electrode(s) at or near the site of the target lesion, may enable the medical professional to perform intraluminal procedures *(e.g.,* an endoscopic submucosal dissection (ESD) procedure) with a higher energy density *(e.g.,* Joules/area) or power density *(e.g.,* Watts/area) at the tissue manipulation site, but with a lower overall amount of energy emitted into or through the surrounding tissues. This may enable the medical professional to employ smaller and more finely controlled instruments, and/or provide a more efficient quantum of energy to the tissue lesion. For example, an intraluminal procedure ordinarily performed using an energy level associated with monopolar electrosurgery may be performed using a surgical system with an energy level typically associated with bipolar electrosurgery.

In various embodiments, the elongate tubular body 110 of any of the surgical systems 100, 200, 300, 400, 500, disclosed herein may further be configured to receive one or more additional medical devices *(e.g.,* scissors, forceps, graspers, scalpels, etc.) through additional working channels *(e.g.,* 116b, 116c). In addition, or alternatively, one or more of the additional working channels may include a lumen for delivery of various fluids, imaging lumens for components of one or more image sensors, vacuum lumens for suctioning air, liquid, or other material from within the body lumen. In addition, or alternatively, a variety of additional medical tools (*e.g.*, scissors, graspers, forceps, knives, needles, balls, hooks, spatulas, etc.) may extend through, or along an outer surface of, the elongate tubular body to further manipulate the tissue wall of the body lumen.

The surgical systems 100, 200, 300, 400, 500, of the present disclosure are not intended to be described or depicted as mutually exclusive embodiments. For example, any or all of the various configurations of return electrodes disposed on or along any of the flexible members, cover, endcap, sleeve and/or expandable member described herein, may be combined into a single surgical system. In addition, any of the return electrodes disclosed herein may be disposed on or along the respective flexible members, cover, endcap, sleeve and/or expandable member of the respective surgical systems in a variety of different numbers, locations, sizes, shapes, patterns and/or orientations.

Non-limiting examples of conductive materials which may comprise the flexible members, surgical device, return electrode(s) and/or conductive elements of any of the surgical systems 100, 200, 300, 400, 500, disclosed herein, may include stainless steel, tungsten alloys, copper, nitinol, titanium, aluminum-based materials, or other suitable conductive materials as are known in the art. Similarly, non-limiting examples of insulative materials, which are described herein, and which may comprise components of the elongate tubular body, working space expanding system, cover, balloon, sheath, distal portion, endcap and/or active electrodes of any of the surgical systems 100, 200, 300, 400, 500, disclosed herein, may include a non-conductive thermoplastic, fluoropolymer, or elastomer such as polytetrafluoroethylene (PTFE), fluorinated ethylene propylene (FEP), silicone, perfluoroalkoxy polymer resin (PFA), ceramic or any other suitable non-conductive material.

All of the devices and/or methods disclosed herein can be made and executed without undue experimentation in light of the present disclosure. While the devices and methods of this disclosure have been described in terms of preferred embodiments, it may be apparent to those of skill in the art that variations can be applied to the devices and/or methods and in the steps or in the sequence of steps of the method described herein.

## Claims

1. A surgical system (100; 200; 300; 400; 500), comprising:
an elongate tubular body (110) comprising a proximal end, a distal end (114) and one or more working channels (116a-c) extending therebetween;
a working space expanding system (120) positioned at a distal portion (112) of the elongate tubular body (110), the working space expanding system (120) movable between a non-expanded insertion position and an expanded position to form an expanded region;
a surgical device (130) disposed within a first working channel (116a) of the elongate tubular body;
at least one return electrode (140a, 140b; 240; 340a, 340b; 440; 540) disposed on an outer surface of the working space expanding system (120); and
a power source configured to deliver energy to the surgical device, and in electrical communication with the at least one return electrode;
wherein the working space expanding system (120) includes first and second flexible members (122, 124) movable between the non-expanded position and the expanded position to form the expanded region; and
wherein a distal portion of the working space expanding system (120) includes an endcap (128), wherein the endcap (128) provides a distal nexus at which a distal end of the first and second flexible members (122, 124) are joined or connected, and wherein the at least one return electrode (140a, 140b; 240; 340a, 340b; 440; 540) is disposed on an outer surface of the endcap (128).

2. The surgical system (100; 200; 300; 400; 500) of claim 1, wherein the surgical device (130) includes an insulated portion (132) and an uninsulated active electrode (134).

3. The surgical system (100; 200; 300; 400; 500) of claim 1 or 2, wherein each of the first and second flexible members (122, 124) includes an insulated portion (123a, 125a) and an uninsulated portion (123b, 125b), and wherein the at least one return electrode (140a, 140b; 240; 340a, 340b; 440; 540) includes a first return electrode (140a) disposed on the uninsulated portion (123b) of the first flexible member (122), and a second return electrode (140b) disposed on the uninsulated portion (125b) of the second flexible member (124).

4. The surgical system (100; 200; 300; 400; 500) of claim 3, wherein the first return electrode (140a) is configured to contact an inner wall of a body lumen (150) at a first location, and the second return electrode (140b) is configured to contact the inner wall of the body lumen (150) at a second location, when the first and second flexible members (122, 124) move to the expanded position.

5. The surgical system (100; 200; 300; 400; 500) of claim 4, wherein the surgical device (130) is configured to contact the inner wall of the body lumen (150) at a third location to deliver energy from the power source, through a portion of tissue of the body lumen (150), and to the first and second return electrodes (140a, 140b).

6. The surgical system (100; 200; 300; 400; 500) of any of claims 1-2, further comprising a cover (326) disposed around a portion of the working space expanding system (120), wherein the at least one return electrode (140a, 140b; 240; 340a, 340b; 440; 540) includes a first return electrode (340a) disposed on an outer surface of the cover (326) at a first location, and a second return electrode (340b) disposed on the outer surface of the cover (326) at a second location.

7. The surgical system (100; 200; 300; 400; 500) of claim 6, wherein the first return electrode (340a) is configured to contact an inner wall of a body lumen (150) at the first location, and the second return electrode (340b) is configured to contact the inner wall of the body lumen (150) at the second location, when the working space expanding system (120) moves to the expanded position.

8. The surgical system (100; 200; 300; 400; 500) of claim 7, wherein the surgical device (130) is configured to contact the inner wall of the body lumen (150) at a third location to deliver energy from the power source, through a portion of tissue of the body lumen (150), and to the first and second return electrodes (340a, 340b).

9. The surgical system (100; 200; 300; 400; 500) of claim 1 or 2, wherein the endcap (128) is configured to contact an inner wall of a body lumen (150) as the first and second flexible members (122, 124) move to the expanded position, to place the at least one return electrode (140a, 140b; 240; 340a, 340b; 440) in contact with the inner wall of a body lumen (150) at a first location.

10. The surgical system (100; 200; 300; 400; 500) of any of claims 1-2, further comprising a sleeve (450) disposed around a distal portion of the elongate tubular body, and wherein the at least one return electrode (140a, 140b; 240; 340a, 340b ; 440) is disposed on an outer surface of the sleeve (450).

11. The surgical system (100; 200; 300; 400; 500) of claim 10, wherein the at least one return electrode (140a, 140b; 240; 340a, 340b; 440)is configured to contact an inner wall of a body lumen at a first location when the working space expanding system (120) moves to the expanded position.

12. The surgical system (100; 200; 300; 400; 500) of claim 11, wherein the surgical device (130) is configured to contact an inner wall of the body lumen (150) at a second location to deliver energy from the power source, through a portion of the tissue of the body lumen (150), and to the at least one return electrode (140a, 140b; 240; 340a, 340b; 440).

13. The surgical system (100; 200; 300; 400; 500) of any of claims 5, 8 or 12, wherein the energy delivered from the surgical device (130) affects a tissue of the body lumen (150).

## Patentansprüche

1. Chirurgisches System (100; 200; 300; 400; 500), aufweisend:
einen länglichen röhrenförmigen Körper (110) mit einem proximalen Ende, einem distalen Ende (114) und einem oder mehreren sich dazwischen erstreckenden Arbeitskanälen (116a-c);
ein Arbeitsraum-Expansionssystem (120), das an einem distalen Abschnitt (112) des länglichen röhrenförmigen Körpers (110) angeordnet ist, wobei das Arbeitsraum-Expansionssystem (120) zwischen einer nichtexpandierten Einführungsposition und einer expandierten Position bewegbar ist, um einen expandierten Bereich zu bilden;
eine chirurgische Vorrichtung (130), die in einem ersten Arbeitskanal (116a) des länglichen röhrenförmigen Körpers angeordnet ist;
mindestens eine Rückelektrode (140a, 140b; 240; 340a, 340b; 440; 540), die auf einer Außenfläche des Arbeitsraum-Expansionssystems (120) angeordnet ist; und
eine Energiequelle, die konfiguriert ist, Energie zu der chirurgischen Vorrichtung zu liefern, und in elektrischer Verbindung mit der mindestens einen Rückelektrode ist;
wobei das Arbeitsraum-Expansionssystem (120) ein erstes und ein zweites flexibles Element (122, 124) aufweist, die zwischen der nichtexpandierten Position und der expandierten Position bewegbar sind, um den expandierten Bereich zu bilden; und
wobei ein distaler Abschnitt des Arbeitsraum-Expansionssystems (120) eine Endkappe (128) aufweist, wobei die Endkappe (128) einen distalen Nexus bereitstellt, an dem ein distales Ende des ersten und des zweiten flexiblen Elements (122, 124) vereinigt oder verbunden sind, und wobei die mindestens eine Rückelektrode (140a, 140b; 240; 340a, 340b; 440; 540) auf einer Außenfläche der Endkappe (128) angeordnet ist.

2. Chirurgisches System (100; 200; 300; 400; 500) nach Anspruch 1, wobei die chirurgische Vorrichtung (130) einen isolierten Abschnitt (132) und eine nichtisolierte aktive Elektrode (134) aufweist.

3. Chirurgisches System (100; 200; 300; 400; 500) nach Anspruch 1 oder 2, wobei jedes der ersten und zweiten flexiblen Elemente (122, 124) einen isolierten Abschnitt (123a, 125a) und einen nichtisolierten Abschnitt (123b, 125b) aufweist, und wobei die mindestens eine Rückelektrode (140a, 140b; 240; 340a, 340b; 440; 540) aufweist: eine erste Rückelektrode (140a), die an dem nichtisolierten Abschnitt (123b) des ersten flexiblen Elements (122) angeordnet ist, und eine zweite Rückelektrode (140b), die an dem nichtisolierten Abschnitt (125b) des zweiten flexiblen Elements (124) angeordnet ist.

4. Chirurgisches System (100; 200; 300; 400; 500) nach Anspruch 3, wobei die erste Rückelektrode (140a) konfiguriert ist, eine Innenwand eines Körperlumens (150) an einem ersten Ort zu kontaktieren, und die zweite Rückelektrode (140b) konfiguriert ist, die Innenwand des Körperlumens (150) an einem zweiten Ort zu kontaktieren, wenn sich das erste und das zweite flexible Element (122, 124) in die expandierte Position bewegen.

5. Chirurgisches System (100; 200; 300; 400; 500) nach Anspruch 4, wobei die chirurgische Vorrichtung (130) konfiguriert ist, die Innenwand des Körperlumens (150) an einem dritten Ort zu kontaktieren, um Energie von der Energiequelle durch einen Gewebeabschnitt des Körperlumens (150) hindurch und zu der ersten und zweiten Rückelektrode (140a, 140b) zu liefern.

6. Chirurgisches System (100; 200; 300; 400; 500) nach einem der Ansprüche 1 bis 2, ferner aufweisend eine Abdeckung (326), die um einen Teil des Arbeitsraum-Expansionssystems (120) herum angeordnet ist, wobei die mindestens eine Rückelektrode (140a, 140b; 240; 340a, 340b; 440; 540) aufweist: eine erste Rückelektrode (340a), die auf einer Außenfläche der Abdeckung (326) an einem ersten Ort angeordnet ist, und eine zweite Rückelektrode (340b), die auf der Außenfläche der Abdeckung (326) an einem zweiten Ort angeordnet ist.

7. Chirurgisches System (100; 200; 300; 400; 500) nach Anspruch 6, wobei die erste Rückelektrode (340a) konfiguriert ist, eine Innenwand eines Körperlumens (150) an dem ersten Ort zu kontaktieren, und die zweite Rückelektrode (340b) konfiguriert ist, die Innenwand des Körperlumens (150) an dem zweiten Ort zu kontaktieren, wenn sich das Arbeitsraum-Expansionssystem (120) in die expandierte Position bewegt.

8. Chirurgisches System (100; 200; 300; 400; 500) nach Anspruch 7, wobei die chirurgische Vorrichtung (130) konfiguriert ist, die Innenwand des Körperlumens (150) an einem dritten Ort zu kontaktieren, um Energie von der Energiequelle durch einen Gewebeabschnitt des Körperlumens (150) hindurch und zu der ersten und der zweiten Rückelektrode (340a, 340b) zu liefern.

9. Chirurgisches System (100; 200; 300; 400; 500) nach Anspruch 1 oder 2, wobei die Endkappe (128) konfiguriert ist, eine Innenwand eines Körperlumens (150) zu kontaktieren, während sich das erste und das zweite flexible Element (122, 124) in die expandierte Position bewegen, um die mindestens eine Rückelektrode (140a, 140b; 240; 340a, 340b; 440) in Kontakt mit der Innenwand eines Körperlumens (150) an einem ersten Ort anzuordnen.

10. Chirurgisches System (100; 200; 300; 400; 500) nach einem der Ansprüche 1 bis 2, ferner aufweisend eine Hülle (450), die um einen distalen Abschnitt des länglichen röhrenförmigen Körpers herum angeordnet ist, und wobei die mindestens eine Rückelektrode (140a, 140b; 240; 340a, 340b; 440) auf einer Außenfläche der Hülle (450) angeordnet ist.

11. Chirurgisches System (100; 200; 300; 400; 500) nach Anspruch 10, wobei die mindestens eine Rückelektrode (140a, 140b; 240; 340a, 340b; 440) konfiguriert ist, eine Innenwand eines Körperlumens an einem ersten Ort zu kontaktieren, wenn sich das Arbeitsraum-Expansionssystem (120) in die expandierte Position bewegt.

12. Chirurgisches System (100; 200; 300; 400; 500) nach Anspruch 11, wobei die chirurgische Vorrichtung (130) konfiguriert ist, eine Innenwand des Körperlumens (150) an einem zweiten Ort zu kontaktieren, um Energie von der Energiequelle durch einen Gewebeabschnitt des Körperlumens (150) hindurch und zu der mindestens einen Rückelektrode (140a, 140b; 240; 340a, 340b; 440) zu liefern.

13. Chirurgisches System (100; 200; 300; 400; 500) nach einem der Ansprüche 5, 8 oder 12, wobei die von der chirurgischen Vorrichtung (130) gelieferte Energie ein Gewebe des Körperlumens (150) beeinflusst.

## Revendications

1. Système chirurgical (100 ; 200 ; 300 ; 400 ; 500), comprenant :
un corps tubulaire allongé (110) comprenant une extrémité proximale, une extrémité distale (114) et un ou plusieurs canaux de travail (116a-c) s'étendant entre elles ;
un système d'expansion d'espace de travail (120) positionné au niveau d'une partie distale (112) du corps tubulaire allongé (110), le système d'expansion d'espace de travail (120) étant mobile entre une position d'insertion non expansée et une position expansée pour former une région expansée ;
un dispositif chirurgical (130) disposé à l'intérieur d'un premier canal de travail (116a) du corps tubulaire allongé ;
au moins une électrode de retour (140a, 140b ; 240 ; 340a, 340b ; 440 ; 540) disposée sur une surface externe du système d'expansion d'espace de travail (120) ; et
une source d'alimentation configurée pour délivrer de l'énergie au dispositif chirurgical, et en communication électrique avec l'au moins une électrode de retour ;
dans lequel le système d'expansion d'espace de travail (120) inclut des premier et deuxième éléments flexibles (122, 124) mobiles entre la position non expansée et la position expansée pour former la région expansée ; et
dans lequel une partie distale du système d'expansion d'espace de travail (120) inclut un capuchon d'extrémité (128), dans lequel le capuchon d'extrémité (128) fournit une jonction distale au niveau de laquelle une extrémité distale des premier et deuxième éléments flexibles (122, 124) sont joints ou connectés, et dans lequel l'au moins une électrode de retour (140a, 140b ; 240 ; 340a, 340b ; 440 ; 540) est disposée sur une surface externe du capuchon d'extrémité (128).

2. Système chirurgical (100 ; 200 ; 300 ; 400 ; 500) selon la revendication 1, dans lequel le dispositif chirurgical (130) inclut une partie isolée (132) et une électrode active non isolée (134).

3. Système chirurgical (100 ; 200 ; 300 ; 400 ; 500) selon la revendication 1 ou 2, dans lequel chacun des premier et deuxième éléments flexibles (122, 124) inclut une partie isolée (123a, 125a) et une partie non isolée (123b, 125b), et dans lequel l'au moins une électrode de retour (140a, 140b ; 240 ; 340a, 340b ; 440 ; 540) inclut une première électrode de retour (140a) disposée sur la partie non isolée (123b) du premier élément flexible (122), et une deuxième électrode de retour (140b) disposée sur la partie non isolée (125b) du deuxième élément flexible (124).

4. Système chirurgical (100 ; 200 ; 300 ; 400 ; 500) selon la revendication 3, dans lequel la première électrode de retour (140a) est configurée pour être en contact avec une paroi interne d'une lumière corporelle (150) au niveau d'un premier emplacement, et la deuxième électrode de retour (140b) est configurée pour être en contact avec la paroi interne de la lumière corporelle (150) au niveau d'un deuxième emplacement, lorsque les premier et deuxième éléments flexibles (122, 124) se déplacent vers la position expansée.

5. Système chirurgical (100 ; 200 ; 300 ; 400 ; 500) selon la revendication 4, dans lequel le dispositif chirurgical (130) est configuré pour être en contact avec la paroi interne de la lumière corporelle (150) au niveau d'un troisième emplacement pour délivrer de l'énergie à partir de la source d'alimentation, à travers une partie de tissu de la lumière corporelle (150), et aux première et deuxième électrodes de retour (140a, 140b).

6. Système chirurgical (100 ; 200 ; 300 ; 400 ; 500) selon l'une quelconque des revendications 1 et 2, comprenant en outre un couvercle (326) disposé autour d'une partie du système d'expansion d'espace de travail (120), dans lequel l'au moins une électrode de retour (140a, 140b ; 240 ; 340a, 340b ; 440 ; 540) inclut une première électrode de retour (340a) disposée sur une surface externe du couvercle (326) au niveau d'un premier emplacement, et une deuxième électrode de retour (340b) disposée sur la surface externe du couvercle (326) au niveau d'un deuxième emplacement.

7. Système chirurgical (100 ; 200 ; 300 ; 400 ; 500) selon la revendication 6, dans lequel la première électrode de retour (340a) est configurée pour être en contact avec une paroi interne d'une lumière corporelle (150) au niveau du premier emplacement, et la deuxième électrode de retour (340b) est configurée pour être en contact avec la paroi interne de la lumière corporelle (150) au niveau du deuxième emplacement, lorsque le système d'expansion d'espace de travail (120) se déplace vers la position expansée.

8. Système chirurgical (100 ; 200 ; 300 ; 400 ; 500) selon la revendication 7, dans lequel le dispositif chirurgical (130) est configuré pour être en contact avec la paroi interne de la lumière corporelle (150) au niveau d'un troisième emplacement pour délivrer de l'énergie à partir de la source d'alimentation, à travers une partie de tissu de la lumière corporelle (150), et aux première et deuxième électrodes de retour (340a, 340b).

9. Système chirurgical (100 ; 200 ; 300 ; 400 ; 500) selon la revendication 1 ou 2, dans lequel le capuchon d'extrémité (128) est configuré pour être en contact avec une paroi interne d'une lumière corporelle (150) lorsque les premier et deuxième éléments flexibles (122, 124) se déplacent vers la position expansée, pour placer l'au moins une électrode de retour (140a, 140b ; 240 ; 340a, 340b ; 440) en contact avec la paroi interne d'une lumière corporelle (150) au niveau d'un premier emplacement.

10. Système chirurgical (100 ; 200 ; 300 ; 400 ; 500) selon l'une quelconque des revendications 1 et 2, comprenant en outre un manchon (450) disposé autour d'une partie distale du corps tubulaire allongé, et dans lequel l'au moins une électrode de retour (140a, 140b ; 240 ; 340a, 340b ; 440) est disposée sur une surface externe du manchon (450).

11. Système chirurgical (100 ; 200 ; 300 ; 400 ; 500) selon la revendication 10, dans lequel l'au moins une électrode de retour (140a, 140b ; 240 ; 340a, 340b ; 440) est configurée pour être en contact avec une paroi interne d'une lumière corporelle au niveau d'un premier emplacement lorsque le système d'expansion d'espace de travail (120) se déplace vers la position expansée.

12. Système chirurgical (100 ; 200 ; 300 ; 400 ; 500) selon la revendication 11, dans lequel le dispositif chirurgical (130) est configuré pour être en contact avec une paroi interne de la lumière corporelle (150) au niveau d'un deuxième emplacement pour délivrer de l'énergie à partir de la source d'alimentation, à travers une partie du tissu de la lumière corporelle (150), et à l'au moins une électrode de retour (140a, 140b ; 240 ; 340a, 340b ; 440).

13. Système chirurgical (100 ; 200 ; 300 ; 400 ; 500) selon l'une quelconque des revendications 5, 8 ou 12, dans lequel l'énergie délivrée à partir du dispositif chirurgical (130) agit sur un tissu de la lumière corporelle (150).
